(19) Europäisches Patentamt

European Patent Office

Office européen des brevets

(11) **EP 3 246 056 A1**

(12) **EUROPEAN PATENT APPLICATION**
published in accordance with Art. 153(4) EPC

(43) Date of publication:
**22.11.2017 Bulletin 2017/47**

(51) Int Cl.:
**A61M 5/158** (2006.01)   **A61M 1/14** (2006.01)

(21) Application number: **16737469.3**

(22) Date of filing: **15.01.2016**

(86) International application number:
**PCT/JP2016/051181**

(87) International publication number:
**WO 2016/114398 (21.07.2016 Gazette 2016/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **16.01.2015 JP 2015007048**

(71) Applicants:
 • **Asahi Kasei Medical Co., Ltd.**
 **Chiyoda-ku**
 **Tokyo 101-8101 (JP)**
 • **Nextier Corporation**
 **Toyohashi-shi, Aichi 441-8107 (JP)**
 • **Togo Medikit Co., Ltd.**
 **Tokyo 113-0034 (JP)**

(72) Inventors:
 • **SASAKI, Masatomi**
 **Tokyo 101-8101 (JP)**
 • **SHINZATO, Toru**
 **Nagoya-shi**
 **Aichi 465-0048 (JP)**
 • **MARUYAMA, Yasuyo**
 **Toyohashi-shi**
 **Aichi 441-8107 (JP)**
 • **SHIGENOBU, Fumitaka**
 **Hyuga-shi**
 **Miyazaki 883-0062 (JP)**

(74) Representative: **dompatent von Kreisler Selting Werner -**
 **Partnerschaft von Patent- und Rechtsanwälten mbB**
 **Deichmannhaus am Dom**
 **Bahnhofsvorplatz 1**
 **50667 Köln (DE)**

(54) **NEEDLE AND METHOD FOR MANUFACTURING SAME**

(57)     The present invention is aimed at suppressing the formation of a pocket in a wall of a puncture route when a needle, being a dull needle, is moved forward along the puncture route. In order to achieve such aim, the invention provides a needle to be inserted into a puncture route extending from skin to a blood vessel under the skin to puncture the blood vessel, wherein: the needle is formed in a tubular shape and has an inclined end face at a tip of the needle, the inclined end face being inclined relative to an axis of the needle, a tip end of the inclined end face defining an unsharp edge; the needle has a flexibility of needle elastic modulus nE = 1-900 N as defined by an equation to be set forth below; a material of the needle is a resin; and the needle is manufactured by injection molding using a ring-shaped gate, wherein

$$\text{the needle elastic modulus } nE = (M_2 - M_1) / (\varepsilon_2 - \varepsilon_1),$$

where:
$M_1$ is a bending moment under a bending strain of 0.0005 in a three-point bending test;
$M_2$ is a bending moment under a bending strain of 0.0025 in the three-point bending test;
$\varepsilon_1$ is a bending strain of 0.0005; and
$\varepsilon_2$ is a bending strain of 0.0025.

**(Cont. next page)**

EP 3 246 056 A1

# FIG.1

**Description**

**Technical Field**

The present invention relates to a needle to be inserted into a puncture route extending from skin to a blood vessel under the skin so as to puncture the blood vessel, as well as to a manufacturing method of such needle.

**Background Art**

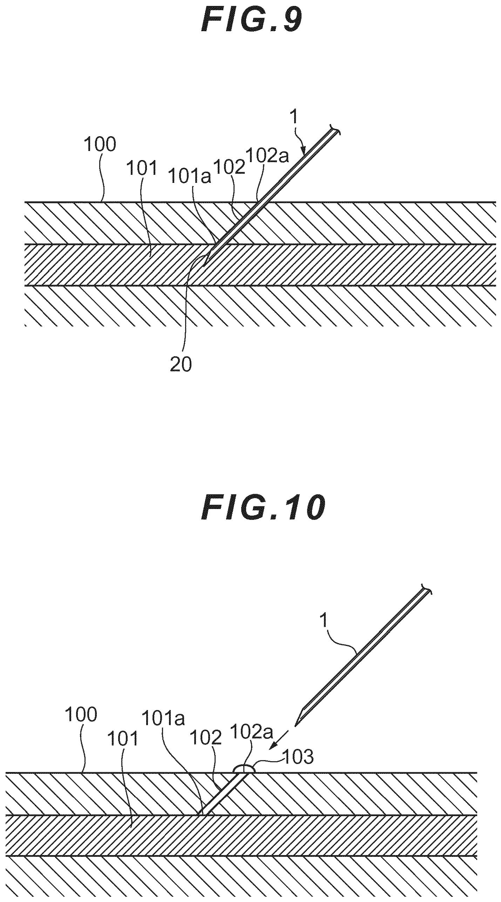

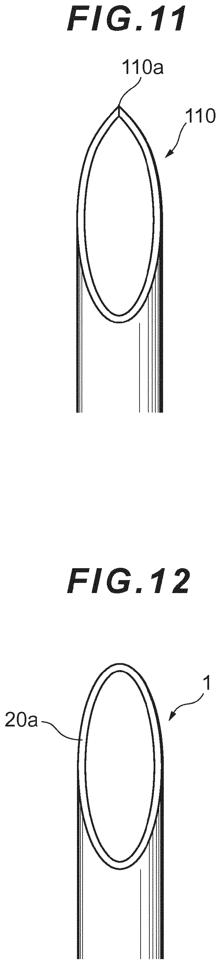

When blood purification treatment, such as dialysis treatment, is performed, a needle is inserted via a patient's skin so as to puncture a blood vessel and blood is collected and returned through the needle. Such puncture operation needs to be performed every time the blood purification treatment is conducted and causes a patient significant pain on each such occasion. Therefore, in order to reduce the pain produced when making a puncture, a so-called "buttonhole puncture method" has been used in recent years. In the buttonhole puncture method, an ordinary needle having high sharpness (an ordinary needle for initial puncture) is used to make a puncture during the first puncture operation to form a puncture route extending from the surface of a body skin 100 to a blood vessel 101, as shown in Fig. 10, and during the second and subsequent puncture operations, a crust 103 is removed and a so-called dull needle 1 having low sharpness is inserted into the puncture route 102 so as to puncture the blood vessel 101. According to this method, the skin 100 does not need to be punctured anew in the second and subsequent puncture operations, and therefore a patient's pain can be reduced. In general, the ordinary needle 110 has a pointed cutting edge 110a at a tip thereof, as shown in Fig. 11, whereas the dull needle 1 has an inclined end face 20, which is formed by obliquely cutting a tubular end, at a tip thereof, and thus has a rounded tip, as shown in Fig. 12.

Citation List

Patent Document

Patent Document 1: JP2009-045124 A

**Summary**

Technical Problem

In the meantime, an operator who makes the puncture operation has to move the dull needle 1 along the axis of the puncture route 102 during the puncture operation. Even a very small deviation of the moving direction of the dull needle 1 from the axial direction of the puncture route 102 causes the tip of the dull needle 1 to contact a wall of the puncture route 102. When the tip of the dull needle 1 contacts the wall of the puncture route 102, the operator feels some resistance on his/her finger pressing the dull needle 1. However, the operator needs to gain significant experience until becoming able to stop pressing the dull needle 1 immediately after feeling such resistance. Thus, in many cases, when the tip of the dull needle 1 contacts the wall of the puncture route 102, a recess is formed in the wall of the puncture route 102.

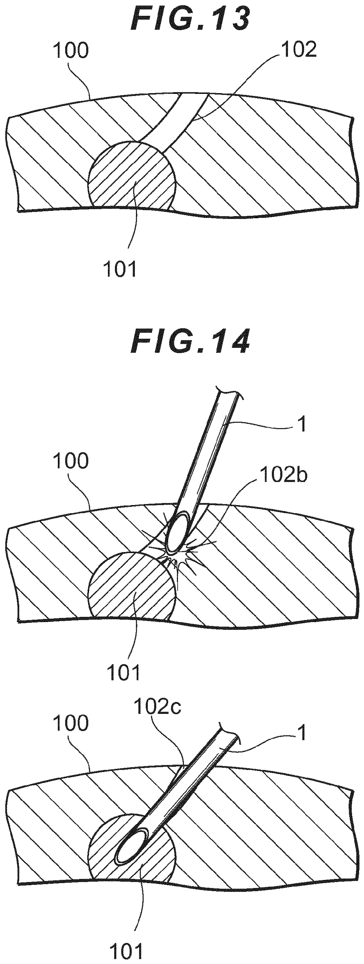

In addition, it is said that even a small shift of the body position of a patient will cause the skin of the patient to be moved or shifted, which will in turn cause a puncture hole 102a, being an entrance of the puncture route 102 on the skin surface, and an entrance of a blood vessel puncture hole 101 a on the exit side of the puncture route 102, to be out of linear alignment with each other. For example, Fig. 13 is a cross-sectional view showing an example in which the position of an arm is slightly shifted as viewed from a fingertip side. Fig. 14(A) shows a state in which a conventional dull needle is inserted into the puncture route 102. These drawings show how the puncture route 102 is skewed and causes the dull needle 1 to contact the wall of the puncture route 102 and form a recess 102b therein. Such a recess is typically called a pocket and such pocket may sometimes be contaminated by bacteria which will serve as a source of infection for the puncture route 102.

When a direction in which the inclined end face of the dull needle 1 is oriented is defined as an upper side, the tip of the dull needle 1 particularly contacts a wall on a lower side of the puncture route 102 and is therefore likely to form a pocket in the wall on the lower side of the puncture route 102. In other words, when the direction in which the inclined end face of the dull needle 1 is oriented is defined as the upper side, the tip of the dull needle 1 is located at the center in a lateral direction (right-and-left direction) and located at the lowermost end in the upper-and-lower direction. Accordingly, if the moving direction of the tip of the dull needle 1 is deviated in the right-and-left direction or deviated upward, a pocket will not be formed in the wall on the lateral side or on the upper side of the puncture route 102, as long as the deviation is small. On the other hand, if the moving direction of the tip of the dull needle 1 is deviated downward, even a small deviation will cause the tip of the dull needle 1 to contact the wall on the lower side of the puncture route 102 and form a pocket in the wall on the lower side of the puncture route 102.

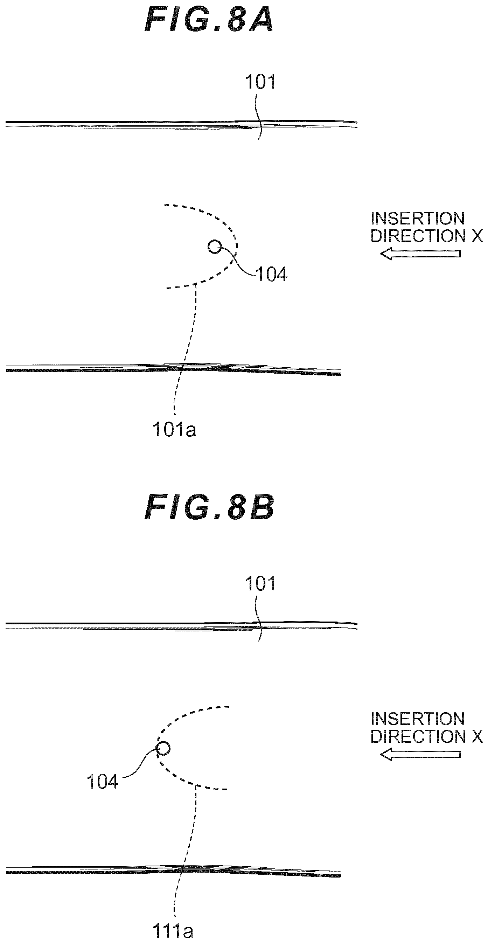

When the dull needle 1 is inserted into the blood vessel 101, a puncture hole 101 a, which opens in a U shape starting from a front side toward a back side in the insertion direction, is formed on the surface of the blood vessel 101, as shown in Fig. 8A. In the second and subsequent puncture operations, the dull needle 1 is inserted into the blood vessel 101 through a trace of the puncture hole 101 a.

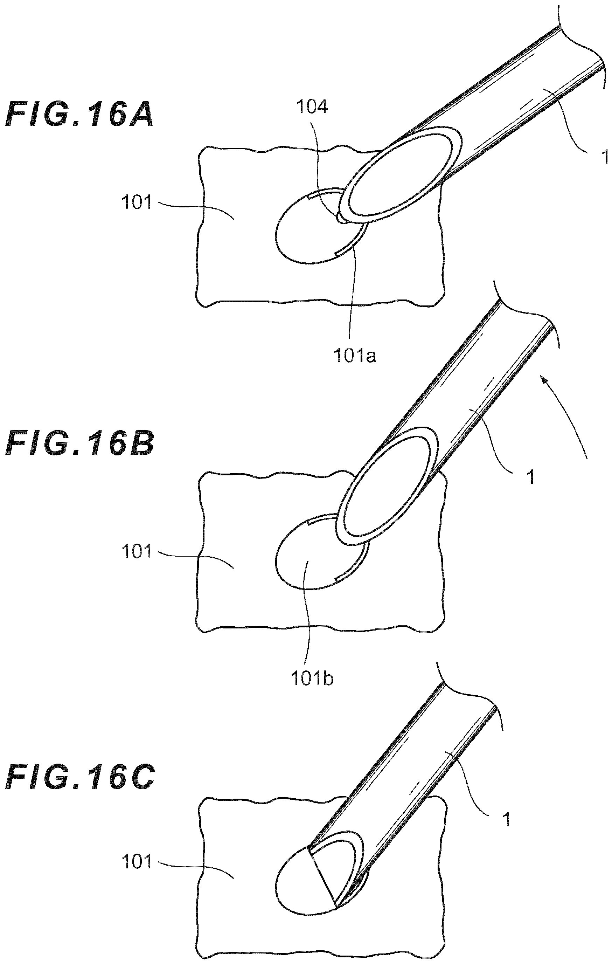

When the dull needle 1 is to be inserted into the blood vessel 101 through the trace of the puncture hole 101 a, the tip of the dull needle 1 is used to search for a so-called optimum contact point 104 in the puncture hole 101 a at which the puncture hole 101 a can be opened by the weakest force, as shown in Fig. 16A.

However, in the conventional puncture hole 101 a, the optimum contact point 104 exists near the bottom of the U shape of the puncture hole 101 a (see Fig. 16A). Thus, the position of the optimum contact point 104 which is searched for by using the tip of the dull needle 1 after forming the puncture route 102 is located on the front side in the insertion direction x of the dull needle 1. Since the optimum contact point 104 is located on the front side, a flap 101 b has to be opened immediately by inserting the dull needle 1 at a relatively large insertion angle, as shown in Fig. 16B. Such an operation causes the resistance in opening the entire joining portion of the puncture hole 101 a to be large and accordingly requires a larger force for inserting the dull needle 1.

Fig. 14(B) shows a cross-sectional view of skin and a blood vessel during dialysis in a situation where the dull needle 1 is being inserted into the blood vessel 101. Since the dull needle 1 is rigid, a gap 102c is produced from the entrance of the puncture route 102 on the skin surface along the puncture route 102. This gap 102c is considered a source of infection in the buttonhole method.

The present invention has been made in view of the above circumstances, and an object of the invention is to provide a needle capable of suppressing the formation of a pocket in a wall in a puncture route when a needle, being a dull needle, is moved forward along the puncture route, as well as a manufacturing method of the needle.

Solution to Problem

A conventional dull needle is a needle from which the sharpness of an ordinary needle is eliminated, and it is therefore made of metal and is rigid. Thus, in a situation in which the moving direction of such conventional dull needle deviates from the axial direction of the puncture route and the tip of the dull needle contacts the wall on the left, right, upper or lower side of the wall of the puncture route (particularly on the lower side of the wall), by the time an operator feels resistance on his/her finger pressing the dull needle, a recess (a so-called "pocket") has already been formed in the wall of the puncture route.

As a result of intensive studies in view of such circumstances, the inventors have found that, by providing the needle with moderate flexibility, it is possible to suppress the formation of a pocket in the wall of the puncture route even when the moving direction of the needle is deviated from the axial direction of the puncture route, and have thereby achieved the present invention. Specifically, the present invention provides a needle to be inserted into a puncture route extending from skin to a blood vessel under the skin to puncture the blood vessel, wherein: the needle is formed in a tubular shape and has an inclined end face at a tip of the needle, the inclined end face being inclined relative to an axis of the needle, a tip end of the inclined end face defining an unsharp edge; the needle has a flexibility of needle elastic modulus nE = 1-900 N, as defined by an equation to be set forth below; a material of the needle is a resin; and the needle is manufactured by injection molding using a ring-shaped gate, wherein

$$\text{the needle elastic modulus nE} = (M_2 - M_1) / (\varepsilon_2 - \varepsilon_1),$$

where:

M_1 is a bending moment under a bending strain of 0.0005 in a three-point bending test;

M_2 is a bending moment under a bending strain of 0.0025 in the three-point bending test;

$\varepsilon_1$ is a bending strain of 0.0005; and

$\varepsilon_2$ is a bending strain of 0.0025.

The present invention also provides a needle to be inserted into a puncture route extending from skin to a blood vessel under the skin to puncture the blood vessel, wherein: the needle is formed in a tubular shape and has an inclined end face at a tip of the needle, the inclined end face being inclined relative to an axis of the needle, a tip end of the inclined end face defining an unsharp edge; a material of the needle is a resin having an elastic modulus in bending of 300-2000 MPa and/or having an elastic modulus in tension of 200-2000 MPa, and/or having a deflection temperature under load (JIS K7191 (ISO 75)) at 0.45 MPa of 70-115°C; and the needle is manufactured by injection molding using a ring-shaped gate.

The present invention further provides a needle to be inserted into a puncture route extending from skin to a blood vessel under the skin to puncture the blood vessel, wherein: the needle is formed in a tubular shape and has an inclined end face at a tip of the needle, the inclined end face being inclined relative to an axis of the needle, a tip end of the inclined end face defining an unsharp edge; the needle has a flexibility of needle elastic modulus nE = 1-900 N as defined by an equation to be set forth below; a material of the needle is a resin; the inclined end face of the needle is free from a cut mark formed by post-treatment; and the needle has a reduced amount of short shot, flash and gate marks, has more uniform wall thickness, and has smoother inner and outer surfaces, as compared to a needle manufactured by injection molding using a pin gate, wherein

$$\text{the needle elastic modulus } nE = (M_2 - M_1) / (\varepsilon_2 - \varepsilon_1),$$

where:

$M_1$ is a bending moment under a bending strain of 0.0005 in a three-point bending test;

$M_2$ is a bending moment under a bending strain of 0.0025 in the three-point bending test;

$\varepsilon_1$ is a bending strain of 0.0005; and

$\varepsilon_2$ is a bending strain of 0.0025.

Examples of the manufacturing methods of a needle made of a resin may include injection molding and extrusion molding. The manufacturing method of a needle using extrusion molding involves the step of creating an opening plane by cutting and, during such process, a cut mark is formed by post-treatment on the inclined end face of the needle. On the other hand, the manufacturing method of a needle using injection molding enables manufacturing of the needle without forming such cut mark. The needle free from the cut mark formed by post-treatment on the inclined end face of the needle may refer to, for example, a needle obtained by injection molding.

The present invention further provides a needle to be inserted into a puncture route extending from skin to a blood vessel under the skin to puncture the blood vessel, wherein: the needle is formed in a tubular shape and has an inclined end face at a tip of the needle, the inclined end face being inclined relative to an axis of the needle, a tip end of the inclined end face defining an unsharp edge; a material of the needle is a resin having an elastic modulus in bending of 300-2000 MPa and/or having an elastic modulus in tension of 200-2000 MPa, and/or having a deflection temperature under load (JIS K7191 (ISO 75)) at 0.45 MPa of 70-115°C; the inclined end face of the needle is free from a cut mark formed by post-treatment; and the needle has a reduced amount of short shot, flash and gate marks, has more uniform wall thickness, and has smoother inner and outer surfaces, as compared to a needle manufactured by injection molding using a pin gate.

The needle may be inserted into the puncture route in which, by inserting a puncture needle with an inclined end face thereof being oriented downward in the puncture route, a puncture mark that opens in an inverted U-shape starting from a back side toward a front side in a puncturing direction is formed in a surface of the blood vessel.

A wall thickness of an arcuate part of the needle may be preferably from 0.02 mm to 0.22 mm.

The wall thickness of an arcuate portion on a tip end side of the inclined end face may be larger than a wall thickness of the remaining arcuate portion, as viewed from a tip side of the needle.

The needle may be formed such that the tip end of the inclined end face is curved upward as viewed from a lateral side of the needle which is horizontally laid still with the inclined end face being oriented upward.

The needle may be formed such that right and left side edges leading to the tip end of the inclined end face are each formed linearly as viewed from an upper side of the needle which is horizontally laid still with the inclined end face being oriented upward.

The needle may be integrally molded with a holding part for holding the needle, and a flow path for a liquid such as blood within the holding part may be tapered such that an inner diameter of the flow path gradually decreases toward the tip of the needle.

The tip end of the inclined end face may define an arcuate edge.

The puncturing method using a puncture needle described in the specification is a method of puncturing a blood vessel by inserting the puncture needle under skin from the surface of the skin, in which the puncture needle is inserted diagonally from the skin surface with the inclined end face of the puncture needle oriented downward toward the skin surface so as to form a puncture mark that opens in an inverted U-shape starting from a back side toward a front side in a puncturing direction.

The manufacturing method of a needle described in the specification is a manufacturing method of a needle to be inserted into a puncture route extending from skin to a blood vessel under the skin to puncture the blood vessel, the method comprising: using a ring-shaped gate as a gate for connecting a runner to the needle, the ring-shaped gate being constituted by a ring part arranged on a resin runner side, a disc part arranged on a needle side, and a connecting part connecting the ring part and the disc part; and performing injection molding in which a resin whose pressure is increased inside the ring part and which is allowed to fill the disc part through the connecting part flows into the needle.

Advantageous Effects of Invention

According to the present invention, it is possible to effectively prevent a pocket, being a potential source of infection, from being formed in a wall of a puncture route and it is therefore possible to provide excellent infection control. It is also possible, during an operation of puncturing a blood vessel through the puncture route, to suppress damage in the puncture route and around the blood vessel. At the same time, the operation of puncturing the blood vessel can be simply performed in a short time. Furthermore, it is possible to alleviate pain involved in the operation of puncturing the blood vessel and is therefore possible to alleviate the burden on the patient. It is also possible to appropriately perform the operation of puncturing the blood vessel, regardless of an operator's experience or technique.

**Brief Description of Drawings**

Fig. 1 is a diagram showing an outline of a configuration of a blood purification treatment device.

Fig. 2 is a perspective view showing an example of a dull needle.

Fig. 3A and 3B are illustrations of a three-point bending test of the dull needle.

Fig. 4 is a graph showing an example of a test force-deflection curve,

Fig. 5 is a top view showing the dull needle having an opening.

Fig. 6 is an illustration showing a state before the dull needle is inserted into skin.

Fig. 7 is an illustration showing a state in which the dull needle is inserted into the skin.

Figs. 8A and 8B are illustrations showing a conventional configuration and an embodiment of a present invention, respectively, of a puncture hole in a blood vessel and an optimum contact point.

Fig. 9 is an illustration showing a state in which the dull needle punctures the blood vessel.

Fig. 10 is an illustration showing a state in which a puncture route is formed.

Fig. 11 is a diagram showing a tip of an ordinary needle.

Fig. 12 is a diagram showing a tip of a conventional dull needle.

Fig. 13 is a cross-sectional view showing an example of a state of a puncture route after the position of an arm is shifted, as viewed from a fingertip side.

Fig. 14 is an illustration showing a puncture state in which an ordinary dull needle is inserted into a puncture route after the position of a body is shifted

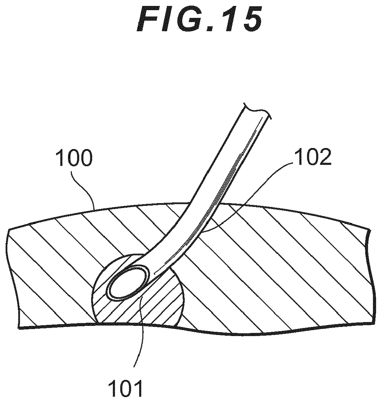

Fig. 15 is an illustration showing a puncture state in which a needle according to the present invention is inserted into a puncture route after the position of a body is shifted.

Figs. 16A, 16B and 16C are illustrations showing a puncture state of a dull needle relative to a conventional puncture hole.

Figs. 17A, 17B and 17C are illustrations showing a puncture state of a dull needle relative to an inverse puncture hole.

Fig. 18 is a diagram showing a conventional molded product using a pin gate.

Fig. 19 is a diagram showing an example of a molded product using a ring gate according to an embodiment of the present invention.

Fig. 20 is an illustration showing an example of a wall thickness of the dull needle as viewed from the tip side thereof.

Figs. 21A and 21B are diagrams showing another example of the shape of an inclined end face of the dull needle.

Figs. 22A, 22B and 22C are a plan view, a side view and a cross-sectional diagram taken along the Z-Z line, respectively, showing the shape of a sample in an Example.

Fig. 23 is a diagram showing a needle and a holding part of a conventional dull needle.

Fig. 24 is a diagram showing an example of an integrally molded product of a needle and a holding part.

Fig. 25 is a diagram showing a device used for measuring a flow rate.

Fig. 26 is an illustration showing a state before a puncture needle is inserted into skin to create an inverse puncture hole.

Figs. 27A and 27B are diagrams showing a conventional configuration and an embodiment of the present invention, respectively, for illustrating differences in (1) a puncture route creation step (first step), and (2) a flap opening step (second step).

**Description of Embodiments**

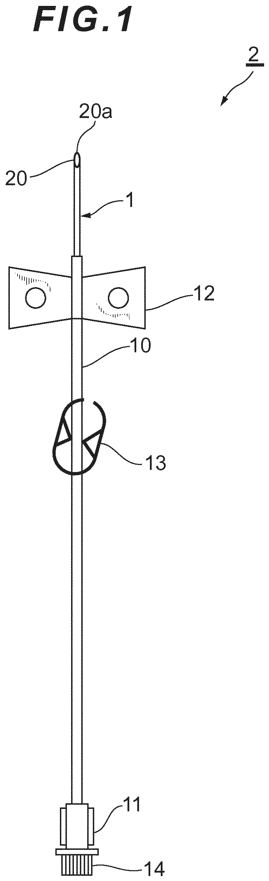

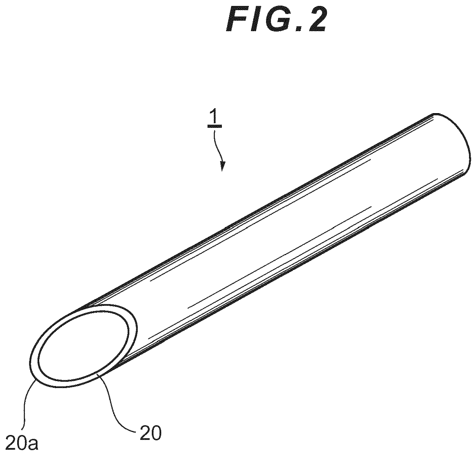

A preferred embodiment of the present invention will be described below with reference to the attached drawings. Fig. 1 is an illustration showing an example of a blood purification treatment device 2 having a dull needle 1, being a needle according to an embodiment of the present invention.
The blood purification treatment device 2 has the dull needle 1 used for making a puncture in a patient during, for example, blood purification treatment, a tube 10 with a front end connected to the dull needle 1, and a connecting part 11 for connecting a rear end of the tube 10 to another tube. A holding part 12 which an operator holds when moving the dull needle 1 is provided at a portion close to the dull needle 1 in the tube 10. A clamp 13 is attached to the tube 10. A cap 14 is fitted to the connecting part 11.
The blood purification treatment device 2 is connected to another tube via, for example, the connecting part 11 and serves as part of a blood purification circuit having a blood purifier (not shown). The blood purification treatment device 2 is attached to a blood collection end segment and a blood return end segment of the blood purification circuit so as to collect or return blood through the dull needle 1 during blood purification treatment. Examples of blood purification treatment may include, without limitation, dialysis treatment, plasma exchange treatment, plasma adsorption treatment and blood component elimination treatment.
The dull needle 1 is formed in a tubular shape, as shown in Fig. 2, and provided with an inclined end face 20 at a tip of the dull needle 1, with the inclined end face 20 being inclined with respect to the axis of the dull needle 1. The inclined end face 20 has a shape formed by, for example, diagonally cutting a tube, and a tip end 20a of the inclined end face 20 defines an unsharp arcuate edge which is curved when viewed from above. It should be noted that the arcuate edge as referred to herein encompasses a flattened elliptical cut edge which appears on a cut plane as a result of diagonally cutting a circular pipe.
The dull needle 1 has flexibility. More specifically, the dull needle 1 preferably has a needle elastic modulus nE, defined by the following equation (1), of 1-900N, more preferably of 10-500N, and even more preferably of 10-400N.

$$\text{Needle elastic modulus } nE = (M_2-M_1) / (\varepsilon_2-\varepsilon_1) \dots (1)$$

$M_1$: Bending moment under a bending strain of 0.0005 in a three-point bending test

$M_2$: Bending moment under a bending strain of 0.0025 in a three-point bending test

$\varepsilon_1$: Bending strain of 0.0005

$\varepsilon_2$: Bending strain of 0.0025

A calculation method of the needle elastic modulus nE will be described in detail below. The needle elastic modulus nE is determined based on JIS K7171 (Plastic - Method of Determining Bending Characteristics).

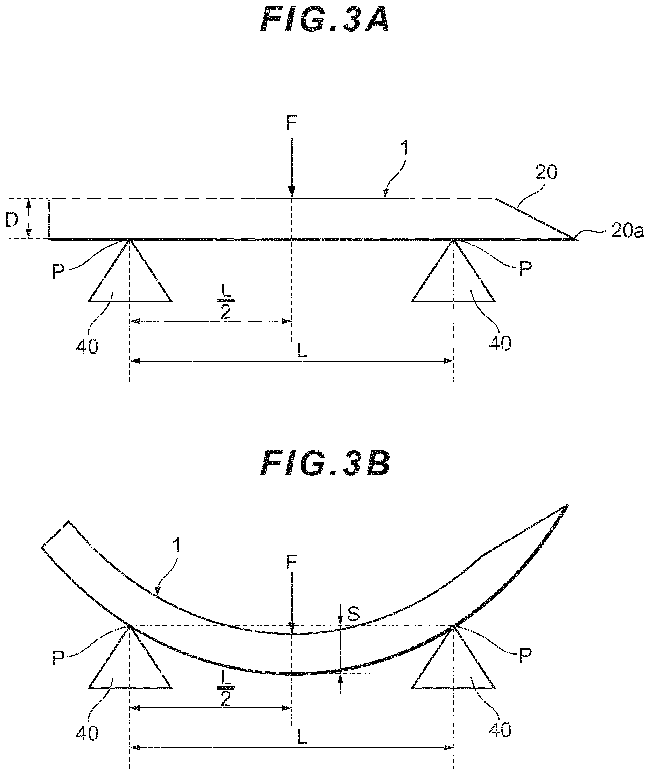

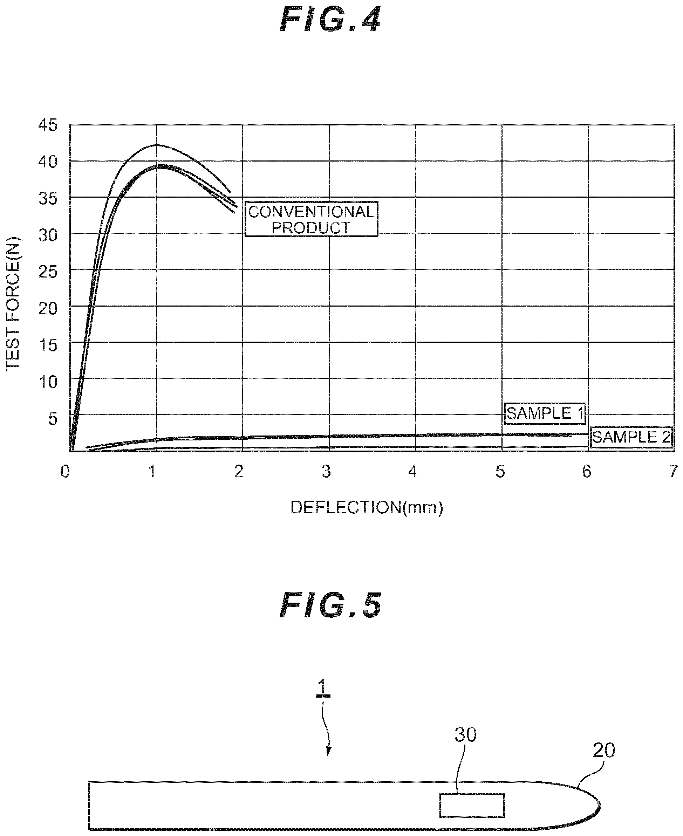

First, in accordance with the three-point bending test defined in JIS K7171, a dull needle 1 is placed on two support tables 40, as shown in Figs. 3(a) and 3(b), test force F is applied onto the center between two supporting points P in the dull needle 1 from a direction perpendicular to the axis of the dull needle 1, and a deflection s is measured. At this time, the distance L between the supporting points is 2 mm and an application speed of the test force F is 2 mm/min. Then the test force F is varied and the deflection s according to each test force F is measured. Based on the measurement results, a test force-deflection curve, as shown in Fig. 4, is prepared.

Meanwhile, deflections $s_1$, $s_2$ under the conditions in which the bending strains $\varepsilon$ of the dull needle 1 are 0.0005 and 0.0025, are calculated using the following equation (2).

$$\text{Bending strain } \varepsilon = (6D/L^2) * s \dots (2)$$

D: Outer diameter of dull needle

L: Distance between supporting points

s: Deflection

Next, based on the deflections $s_1$, $s_2$ calculated by equation (2) and the above-mentioned test force-deflection curve, test forces $F_1$, $F_2$ from when the bending strain $\varepsilon$ is 0.0005 and 0.0025, are determined. Based on the test forces $F_1$, $F_2$, bending moments $M_1$, $M_2$ under the respective bending strains $\varepsilon$, are determined using the following equation (3).

$$\text{Bending moment } M = LF/4 \dots (3)$$

F: Test force

L: Distance between supporting points

Lastly, the needle elastic modulus nE is determined by substituting bending moment M1 under the bending strain of 0.0005, bending moment M2 under the bending strain of 0.0025, and bending strains $\varepsilon_1$ (0.0005), $\varepsilon_2$ (0.0025) in equation (1).

It should be noted that when a dull needle 1 having an opening (back eye) 30 in a lower part, as shown in Fig. 5, is used in the above test, the positions of the supporting points P should be located away from the opening 30.

Examples of the materials of the flexible dull needle 1 may include a thermoplastic resin. For example, the material of the dull needle 1 may employ a resin having an elastic modulus in bending of 1-2500 MPa, preferably of 300-2000 MPa, and more preferably of 500-1800 MPa. The elastic modulus in tension of such material is preferably 200-2000 MPa and more preferably 400-1800 MPa. The deflection temperature under load (JIS K7191 (ISO 75)) at 0.45 MPa of such material is preferably 50-120°C and more preferably 70-115°C. The material of the dull needle may satisfy all or part of the above-mentioned elastic modulus in bending, elastic modulus in tension and deflection temperature under load. In order to enhance the manufacturing efficiency, a resin having good injection moldability or good extrusion moldability is preferable. A resin to be used for injection molding preferably has an MFR (Melt Flow Rate) of 1-60g/10 min and more preferably of 8-60 g/10 min. A resin to be used for extrusion molding preferably has an MFR (Melt Flow Rate) of 0.1-10 g/10 min and more preferably of 0.5-8.5 g/10 min. Examples of preferable materials of the dull needle 1 may include polypropylene, polyethylene, polytetrafluororethylene, ABS resin (Acrylonitrile Butadiene Styrene) and polycarbonate.

The outer diameter of the dull needle 1 is preferably about 0.4-2.5 mm and the inner diameter of the dull needle 1 is preferably about 0.35-2.4 mm. The total length of the dull needle 1 is preferably about 10-120 mm.

With regard to a manufacturing method of the above-mentioned dull needle 1, high-speed and high-pressure injection molding is preferable in order to achieve a needle with a smaller wall thickness and lighter weight, as compared to a typical injection molding method. In addition, a ring gate 50 is preferably used as a gate for connecting a runner 200 and the dull needle 1. If a conventional pin gate 150 is used as the gate, it provides good mold releasability; however, a filling speed is likely to vary between a position close to the gate and a position distant from the gate, and the variation in the filling speed may disadvantageously cause short shot in a portion with insufficient filling and cause flash in a portion with excessive filling. In addition, if injection molding using the pin gate 150 is performed in order to manufacture a dull needle 1 with a thin wall thickness, as in the present embodiment, cooling timing is likely to be shifted from its expected time and extreme warping tends to be generated in the dull needle 1. In addition, if the pin gate 150 is used, the dull needle 1 may sometimes have a gate mark thereon after being released from the gate within a mold. On the other hand, the present embodiment employs a ring gate 50 that is constituted by: a ring 53 in which the flow pressure and the flow of the resin are adjusted; a connecting pipe 52; and a disc 51. With such configuration, the resin whose pressure is increased in the ring 53 and which is allowed to fill disc 51 through the connecting pipe 52 immediately flows into the dull needle 1. Therefore, it is possible to perform filling despite the thin wall of the dull needle 1 of the present embodiment and is also possible to suppress variation in filling in a circumferential direction so as to thereby prevent the undesirable generation of flash. The inclined end face at the tip of the injection-molded dull needle 1 is formed during molding and is free from a cut mark formed by post treatment.

In order for the ring gate 50 to maintain the pressure, speed and uniform fluidity of the resin flowing into the dull needle 1, the outer diameter of the disc 51 is preferably 5.0-7.0 mm, the wire shape of the ring 53 is preferably 1.0-2.0 mm, and the outer diameter of the ring 53 is preferably 11.0-13.0 mm. The number of connecting pipes 52 connecting the ring 53 and the disc 51 may preferably be 2 or 3. In order to adjust the flow viscosity of the resin, the mold temperature of the dull needle 1 is preferably maintained at 80-95°C and more preferably at 90°C. In order to achieve uniformly thin walls, the nozzle temperature is preferably set, for precise adjustment of the resin viscosity, to 220-240°C (front part), 210-230°C (middle part) and 200-220°C (rear part), and more preferably to 230°C (front part), 220°C (middle part) and 210°C (rear part).

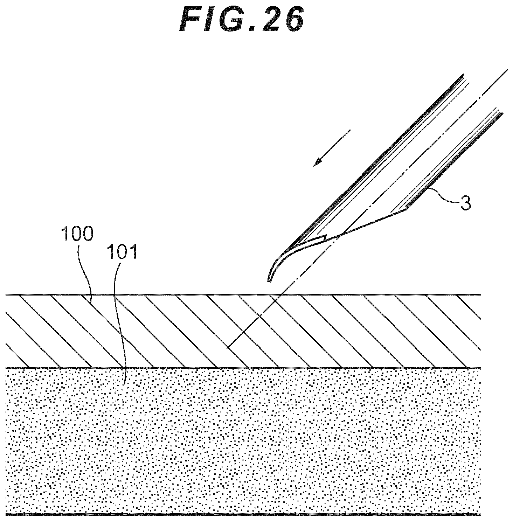

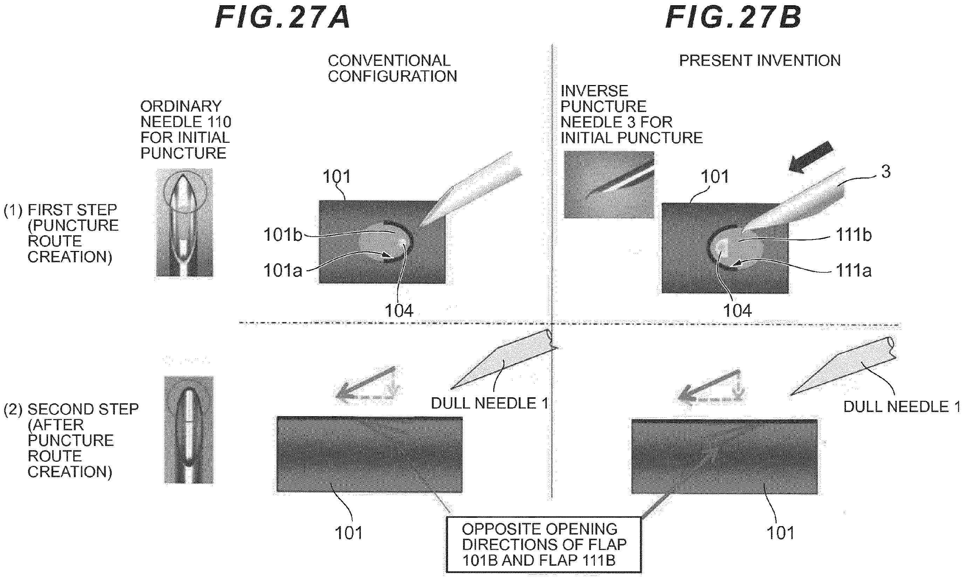

The following description will describe an example of an operation of puncturing a blood vessel 101 using the dull needle 1 configured as described above. Before this puncture operation is performed, a puncture using an initial needle will have already been performed and a puncture route 101 extending from a surface of skin 100 to the blood vessel 101 will have already been formed at a shunt of a patient, as shown in Fig. 10. A crust 103 is formed at an entrance of the puncture route 102. As a method of forming the puncture route 102, for example, a puncture needle (an inverse puncture needle for initial puncture) 3 is inserted into the skin 100 at a certain angle from diagonally upward relative to the surface of the skin 100 with its inclined end face being oriented downward (oriented toward the surface of the skin 100), as shown in Figs. 26 and 27. When the puncture needle 3 is inserted subcutaneously from the skin 100, the puncture needle 3 reaches a surface of the blood vessel 101, opens a puncture hole 111 a and enters the blood vessel 101. At this time, since the puncture hole 111 a is formed on the surface of the blood vessel 101 so as to open in an inverted U-shape, starting from a back side toward a front side in an insertion direction (puncture direction) of the dull needle 1, as shown in Fig. 8B, a so-called optimum contact point 104 is formed on the rear side in the insertion direction. Specifically, since the insertion direction of the dull needle 1 coincides with the opening direction of the puncture hole (unlike the conventional configuration in which the puncture hole 101 a is formed on the surface of the blood vessel 101 so as to open in a U-shape starting from the front side toward the back side in the insertion direction of the dull needle, as shown in Fig. 8A), an angle of insertion of the dull needle into the blood vessel is relatively small and a resistance against the insertion of the dull needle is thus small, and it is therefore possible for the dull needle 1 to be inserted into the blood vessel with relatively small force. The blood purification treatment device 2 having such dull needle 1 is taken out of a sterile bag and connected to a blood purification circuit.

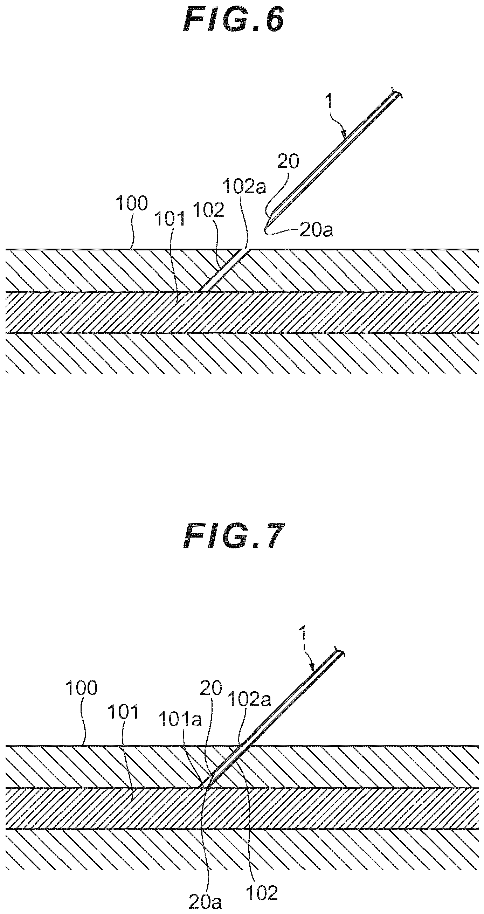

During the puncture operation using the dull needle 1, the crust 103 on the puncture route 102 is first removed by the dull needle 1, etc., as shown in Fig. 6, so that the puncture hole 102a opens in the skin 100. Next, the dull needle 1 is inserted from the puncture hole 102a into the puncture route 102. During this operation, since the dull needle 1 has moderately flexibility, it follows the bending or displacement of the puncture route 102. In addition, even if the dull needle 1 deviates from the axial direction of the puncture route 102 and contacts the wall of the puncture route 102, the dull needle 1 is bent by such small resistance. As a result, the operator can feel the resistance resulting from such bending on his/her finger pressing the dull needle 1 and can therefore immediately stop pressing the dull needle1.

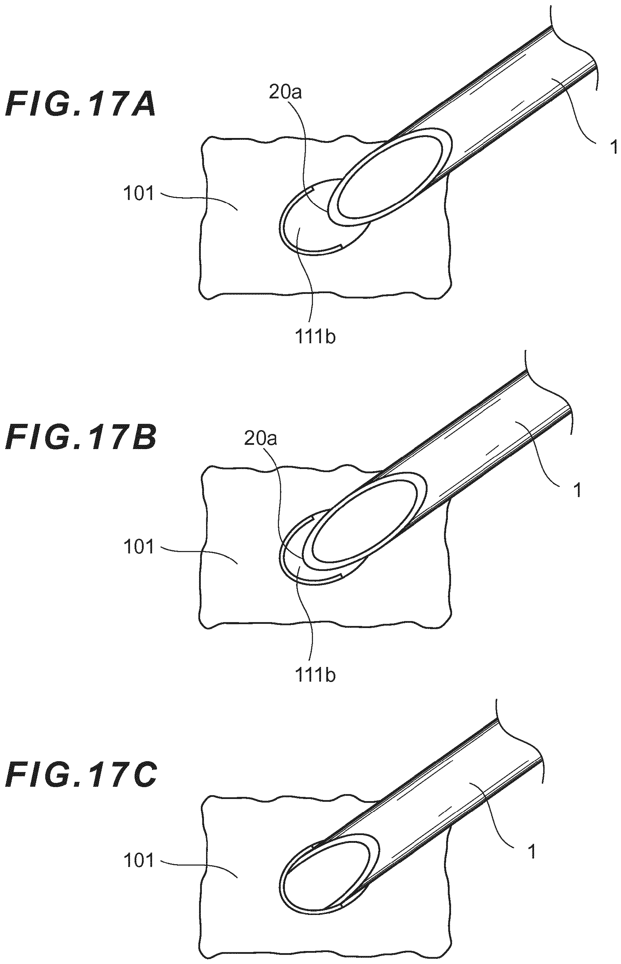

If the dull needle 1 is further moved forward into the back side of the puncture route 102, the tip end 20a of the dull needle 1 reaches the closed U-shaped puncture hole 101 a in the surface of the blood vessel 101. Then, while pressing the tip end 20a of the dull needle 1 against the puncture hole 101 a, the operator searches for the optimum contact point 104 of the puncture hole 101 a, as shown in Figs. 8A and 8B. At this time, since the dull needle 1 has moderate flexibility, the dull needle 1 can flexibly accommodate the positional displacement of the optimum contact point 104. As shown in Figs. 17A to 17C, the tip end 20a of the dull needle 1 is applied onto the optimum contact point 104. Since the optimum contact point 104 is located on the back side in the puncture direction, the insertion angle of the dull needle 1 into the blood vessel 101 does not have to be changed and the resistance of the insertion can therefore be reduced. Specifically, the entire joining portion of the puncture hole 111 a does not have to be opened immediately and only a joining portion of the puncture hole 111 a which the tip of the dull needle 1 contacts should be opened, and it is therefore possible to easily insert the dull needle 1 into the blood vessel 101 with smaller force. When the tip end 20a of the dull needle 1 is applied onto the optimum contact point 104, the puncture hole 111 a opens from this optimum contact point 104, a flap 111 b is pressed downward, and the dull needle 1 punctures the blood vessel 101, as shown in Fig. 9. Then, blood is collected and returned through the dull needle 1 and blood purification treatment is performed using the blood purification circuit.

According to the present embodiment, since the dull needle 1 has moderate flexibility, the dull needle 1 follows the bending or displacement of the puncture route 102 which is generated during the puncture operation. In addition, even if the dull needle 1 contacts the wall of the puncture route 102, the operator can immediately feel the contact and stop pressing the dull needle 1 and it is therefore possible to suppress the formation of a so-called "pocket" in the puncture route 102. The dull needle 1 can also accommodate a slight positional displacement of the optimum contact point 104 and the searching ability of the needle can be improved. Accordingly, it is possible to prevent infections or damage from occurring around the puncture route 102. In addition, the operation of puncturing the vessel 101 can be performed easily in a short time. Moreover, it is possible to alleviate the pain involved in the operation of puncturing the blood vessel and is therefore possible to alleviate the burden on a patient. Furthermore, the operation of puncturing the blood vessel can be performed regardless of the operator's experience or technique.

In the above-mentioned embodiment, the dull needle 1 has moderate flexibility and the needle elastic modulus nE thereof is 1-900 N. A needle elastic modulus nE of greater than 900 N would cause the dull needle 1 to have too much rigidity and would degrade its ability to follow the puncture route. On the other hand, a needle elastic modulus nE of less than 1 N would cause the force for pressing the flap 101 b downward to be too small to disjoin the flap 101 b from the surface of the blood vessel. In such case, even if the moving direction of the dull needle 1 does not deviate from the axial direction of the puncture route 102, the dull needle 1 may still possibly be bent due to friction between the dull needle 1 and the puncture route 102.

According to the above-mentioned embodiment, since the dull needle 1 has flexibility, the wall thickness of the dull needle 1 can be reduced and the inner diameter thereof can therefore be increased for the dull needle 1 having the same gauge (the same outer diameter) and, accordingly, the pressure loss can be reduced and the flow rate can be increased.

Although a conventional metallic dull needle has to be disposed of in landfills, the dull needle 1 made of a resin may be disposed of by incineration and such dull needle 1 is excellent in terms of being an environmental and antipollution measure. In addition, resin is easily moldable and the manufacturing cost can be reduced by using such resin. The dull needle 1 made of a resin can also be formed into a complicated shape which is suitable for making a puncture through the puncture route 102.

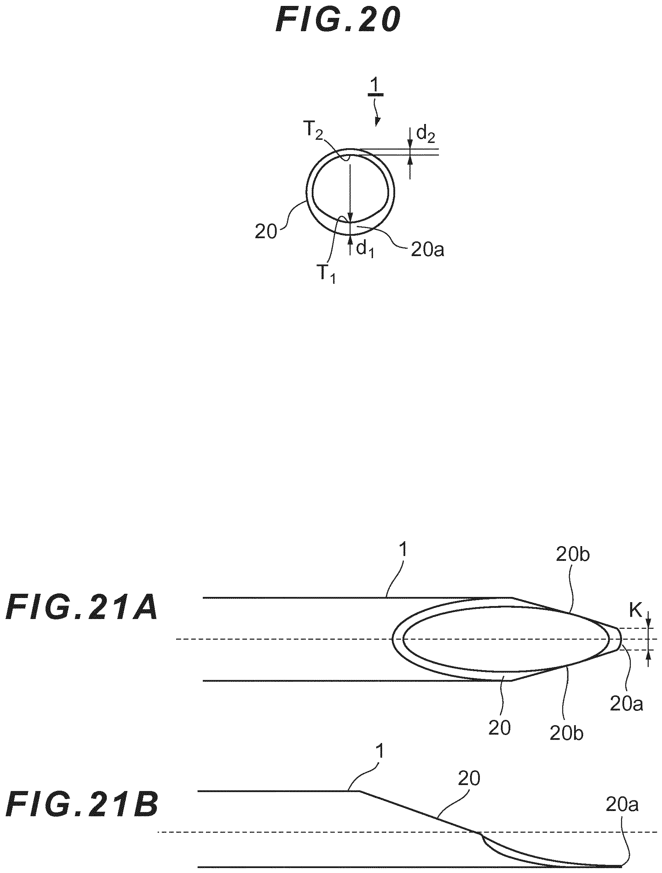

In the above embodiment, the dull needle 1 may be formed such that the wall thickness of an arcuate portion T1 on the tip end 20a side of the inclined end face 20 as viewed from the tip side of the dull needle 1 may be larger than the wall thickness of the remaining arcuate portion T2, as shown in Fig. 20. The wall thickness d1 of the arcuate portion T1 on the tip end side is preferably larger, by 0.0.1-0.5 mm, than the wall thickness d1 of the arcuate portion T2 which opposes the tip end 20a (on the opposite side). By forming the tip end 20a side so as to have a larger wall thickness to thereby enhance its strength, it is possible to cause the tip end 20a of the inclined end face 20 to be resistible against the load to be applied thereto during the insertion of the dull needle 1 while forming the remaining arcuate portion T2 so as to have a smaller wall thickness. By forming the remaining arcuate portion T2 so as to have a smaller wall thickness, it is possible to increase the inner diameter of the dull needle 1 and thus increase the flow rate of the flow through the dull needle 1. As a result, time taken for the blood purification treatment performed by inserting the dull needle 1 can be shortened. Furthermore, the fluidity of resin during the injection molding can be improved by providing a thick-wall portion T1, and manufacturing efficiency can be improved with the decreasing wall thickness of the arcuate portion T2

The wall thickness of the arcuate portion T2 other than the tip end 20a may be 0.02-0.22 mm and preferably 0.08-0.15 mm. A wall thickness above 0.22 mm would cause the outer diameter of the dull needle 1 to be increased in order to secure a sufficient flow rate, which would disadvantageously increase the burden or pain on a patient during a puncture operation. On the other hand, a wall thickness of below 0.02 mm would reduce the strength of the dull needle 1 and thus cause the dull needle 1 to be prone to buckling or breakage.

A cylinder part of the above-mentioned dull needle 1 has to be configured to have a needle elastic modulus within the above-mentioned range so as to have moderate flexibility in order to follow the puncture route (a subcutaneous tunnel) 102. However, the needle tip end 20a may have a different specification. Since the needle tip end 20a needs high strength in order to press the puncture hole 102a outward and the strength of the needle tip end 20a is structurally lowered at the opening thereof, the needle tip end 20a and the needle cylinder part (the cylinder part other than the inclined end face 20 in the dull needle 1) 1a may have different specifications. For example, a harder resin may be used for the needle tip end 20a, whereas a moderately flexible resin may be used for the needle cylinder part 1a so as to increase the strength of the needle tip end 20a. Alternatively, the wall thickness of the needle tip end 20a may be made larger than that of the needle cylinder part 1a. In such configuration, the wall thickness of the needle tip end 20a on the opposite side of the opening end may preferably be made larger in consideration of the flow rate inside the needle. In such configuration, the needle tip end 20a may preferably refer to an area extending from 0.5 mm to 6.0 mm from the tip of the needle.

Although a connecting portion between the dull needle 1 and the holding part 12 receives force during insertion of the dull needle 1, the connecting portion does not have to follow the puncture route (subcutaneous tunnel) 102 and its specification may therefore be different from the remaining needle cylinder part. For example, only an end portion of the needle cylinder part may be formed in a tapered manner so that the wall thickness thereof is gradually increased toward the connecting portion 1b between the needle cylinder part 1a and the holding part 12 in order to increase the strength of the end portion of the needle cylinder part.

As viewed from a lateral side of the dull needle 1 which is horizontally laid still with the inclined end face oriented upward, the tip end may be curved upward. In a configuration in which the tip end of the dull needle 1 is curved upward, even if the moving direction of the tip of the dull needle 1 is slightly deviated downward with respect to the long axis of the puncture route 102, a pocket will still not be formed in the wall on the lower side of the puncture route 102. In addition, in order to prevent the strength of the tip end from decreasing in the configuration in which the end portion of the dull needle 1 is curved upward, the wall thickness of the arcuate portion T1 on the tip end 20a side of the inclined end face 20 is preferably larger than the wall thickness of the remaining arcuate portion T2, as viewed from the tip side. The portion to be curved upward in the end portion of the dull needle 1 may preferably extend from 0.1 % to 50% of the inner diameter of the dull needle 1, and particularly preferably from 1 % to 20%. The end portion of the dull needle 1 which is curved upward may preferably be oriented toward the center line of the inner diameter of the dull needle 1, because such orientation will lower the risk of formation of a pocket in the wall of the puncture route. If more than 50% of the end portion of the dull needle 1 is curved upward, operability in puncturing the surface of the skin and the surface of the blood vessel will be disadvantageously degraded.

Although the inclined end face 20 of the dull needle 1 has an elliptical shape in the above embodiment, it may have other shapes. For example, as shown in Fig. 21A, as viewed from an upper side of the dull needle 1 which is horizontally laid still with the inclined end face 20 oriented upward, right and left side edge portions 20b leading to the tip end 20a may each be formed in a linear manner. In such configuration, a tip width K of the thinned tip end 20a is preferably 0.01-1.5 mm and particularly preferably 0.02-0.6 mm. It should be noted that Fig. 21B is a side view of the dull needle 1 of Fig. 21A.

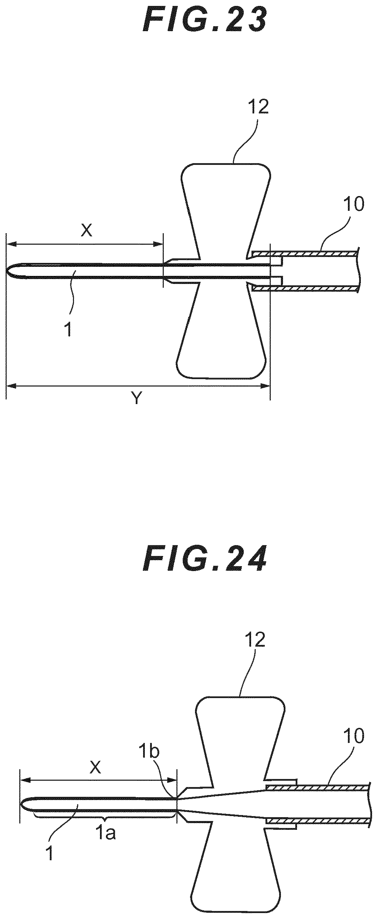

The dull needle 1 and the holding part 12 are joined to each other by fitting, as shown in Fig. 23. Thus, in order to secure an effective length X of the dull needle 1, a total length Y, corresponding to the sum of the effective length X and a fitting length, is necessary. In the embodiment shown in Fig. 24, by integrally molding the dull needle 1 having a holding function (i.e., by molding the dull needle 1 and the holding part 12 together as a single molded product or as a single article) so as to eliminate a step in a liquid flow path 12a in a pipe extending from the tube 10 to the dull needle 1, antithrombogenicity during blood circulation can be improved and the flow rate can be increased. A configuration in which a step is eliminated in a bore extending from the dull needle 1 to the tube 10, or the bore is radially expanded (i.e., the bore is gradually expanded from the inner diameter of the dull needle 1 to the inner diameter of the tube 10 in a tapered manner, as shown in Fig. 24) is more preferable because such configuration can improve antithrombogenicity.

When a polypropylene resin or a polyethylene resin is used in adhesion between the dull needle 1 and the holding part 12 or between the tube 10 and an integrally molded product of the dull needle 1 and the holding part 12, a method of performing plasma treatment and/or primer treatment and using an ultraviolet curable resin is a simple and economical method which provides a high adhesion strength. For example, the ultraviolet curable resins may be of epoxy cationic polymerization or acrylate radical polymerization and, among the latter, epoxy modified acrylate, silicone modified acrylate and urethane modified acrylate are particularly suitable.

Although preferred embodiments of the present invention have been described above with reference to the attached drawings, the present invention is not limited to such embodiments. A person skilled in the art could obviously conceive of various changes and modifications within the scope of the ideas set forth in the claims, and such changes and modifications should be interpreted as being encompassed in the technical scope of the present invention.

For example, although the dull needle 1 is used in a blood purification treatment in the above embodiment, the dull needle 1 may be used in any form of treatment as long as it is used in a so-called "buttonhole puncture method." For example, the present invention is also applicable to a dull needle which is used in administration of a drug using an injection syringe or in the collection of blood. Although the dull needle 1 may be made of a resin in the above embodiment, other materials such as ceramics and metals and other shapes may be employed, as long as a specific needle elastic modulus nE can be secured.

Example 1

(Evaluation Test 1)

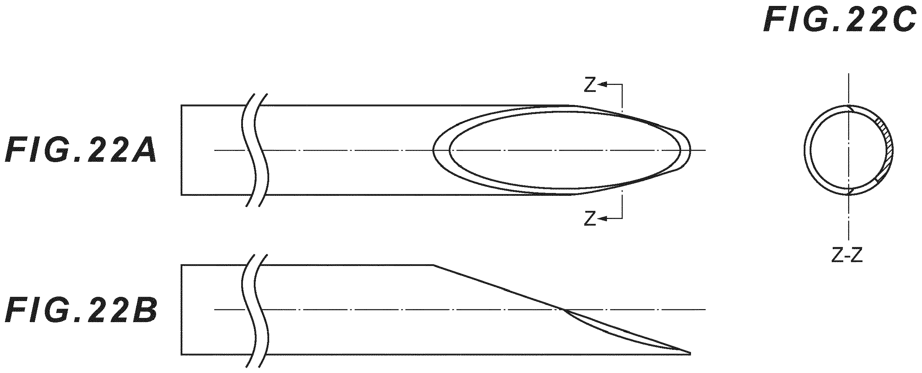

A needle elastic modulus nE was calculated for each sample of the dull needle 1 according to the present invention and a conventional dull needle. Sample 1 is a tubular dull needle made of Teflon (registered trademark) and having an outer diameter of 1.7 mm and an inner diameter of 1.3 mm. Sample 2 is a tubular dull needle made of polypropylene and having an outer diameter of 1.5 mm and an inner diameter of 1.1 mm. Sample 3 (comparative example) is a tubular dull needle made of stainless steel and having an outer diameter of 1.5 mm and an inner diameter of 1.2 mm. The shape of Samples 1 and 2 is shown in Figs. 22A to 22C and the shape of Sample 3 is shown in Fig. 2. Samples 1 and 2 have the same shape, although they are made of different materials.
Samples 1 to 3 were subjected to a three-point bending test defined by JIS K 7171, under the conditions shown in Table 1.

Table 1

| Humidity | 23 $\pm$ 2°C | Humidity | 50 $\pm$ 5% RH |
|---|---|---|---|
| Test machine type | AG-X (manufactured by Shimadzu Corporation) | Load cell capacity | 50 N |
| Distance between supporting points | 2 mm | Speed | 2 mm/min |

As described in the above embodiment, a test force-deflection curve was prepared based on the results of the three-point bending test and each needle elastic modulus nE was calculated using Equations (1) to (3). The results are shown in Table 2.

Table 2

| Sample | Needle elastic modulus (N) |
|---|---|
| Conventional product | 21015 |
|  | 21357 |
|  | 21169 |
|  | 21237 |
| Average | 21194 |
| Standard deviation | 143 |
| Sample 1 | 363 |
|  | 361 |
|  | 359 |
|  | 364 |
| Average | 361 |
| Standard deviation | 2 |
| Sample 2 | 93 |
|  | 90 |
|  | 93 |
|  | 88 |
| Average | 91 |
| Standard deviation | 2 |

The needle elastic modulus nE of the dull needle 1 according to the present invention was much lower than that of the conventional dull needle and exhibited flexibility.

Manufacturing methods of molded products were evaluated.

(Evaluation Test 2)

A dull needle 1 molded using a ring gate 50 was compared to a dull needle 1 molded using a conventional pin gate 150. In the molding using the ring gate 50, a high-speed injection molding machine (manufactured by NISSEI PLASTIC INDUSTRIAL CO., LTD.) was used under injection conditions of 6-speed control, limit pressure of 1700 kgf/cm$^2$, holding pressure of 30 kgf/cm$^2$, mold temperature of 90°, and nozzle temperatures of 230° (front part), 220° (middle part) and 210° (rear part).

Comparative Example 1

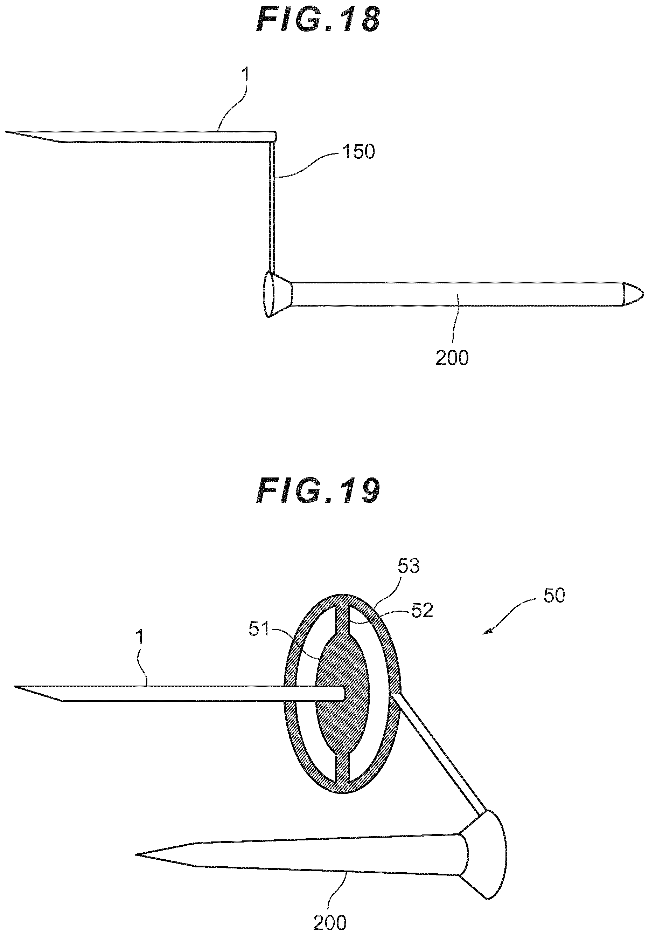

A mold for producing a molded product using the pin gate 150 shown in Fig. 18 was designed and subjected to high-speed injection molding. The filling speed of resin varied between a position close to the gate and a position distant from the gate, and excessive warping was generated in the molded product due to short shot (insufficient filling), flash (excessive filling), and a shift in the cooling timing of the resin. In addition, the method using the pin gate 150 caused a gate mark to be formed and caused the dull needle 1 to have surface irregularity.

Example 2

A mold for producing a molded product using the ring gate 50 shown in Fig. 19 was designed and subjected to high-speed injection molding. From the resulting molded product, a dull needle 1 having a wall thickness of 1 mm, an outer diameter of 1.47 mm and a length of 41 mm could be prepared. The resulting dull needle 1 was free from short shot, flash and gate mark, and had a uniform wall thickness and smooth inner and outer surfaces. This was achieved by a configuration in which the pressure of the resin flowing from the runner 200 was increased in the ring gate 50 and the resin was allowed to immediately fill a needle portion. Such configuration could achieve filling despite the thin wall thickness of the dull needle 1, and was able to suppress variations in filling in the circumferential direction and prevent undesirable generation of flash.

A measurement of the flow rate was conducted on an integrally molded product of the dull needle 1 and the holding part 12.

(Evaluation Test 2)

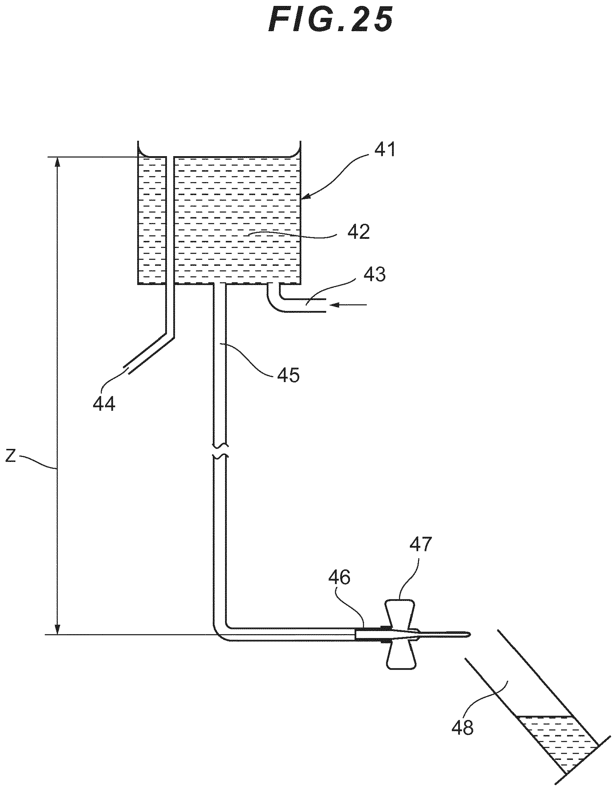

Fig. 25 shows a flow rate measuring device. A constant water level bath 41 has an overflow pipe 44 and is constantly replenished with a measurement fluid by an intake 43 so that a static water head pressure Z is maintained in a constant manner. A sample 47 was attached to a fitting part 46. The fluid after flowing for a given period of time was collected in a collection/measurement container 48 and the amount of fluid was measured. The flow rate was measured for the integrally molded product of the dull needle 1 and the holding part 12 in Fig. 24 (Sample 4) and for a product obtained by fitting the dull needle 1 with the holding part 12 in Fig. 23 (Sample 5). The dimensions of the samples of the dull needle 1 subjected to this measurement were as follows: outer diameter = 1.5 mm, X = 28 mm and Y = 41 mm. Tap water was used as the measurement fluid and the measurement was conducted at room temperature.

Table 3

| Evaluation sample | Flow rate (ml/min) |
|---|---|
| Integrally-molded product according to the present invention (Sample 4) | 303 ± 3 |
| Conventional fitting-type product (Sample 5) | 201 ± 2 |

The flow rate of the integrally-molded product according to the present invention was increased by 51% as compared to the flow rate of the conventional product.

(Evaluation Test 2)

Evaluation test 2 evaluated the puncturing performance of the dull needle 1 using two adult mixed-breed dogs (weight: 16-25 kg) under the following experiment conditions: after intramuscular injection of Cectarol (1 mg/kg) and antrobin sulfate (0.05 mg/kg), endotracheal intubation was conducted and anesthesia was maintained using ketamine at the rate of 40 mg/kg/hr.

A puncture needle was used to prepare, in advance, a puncture route (subcutaneous tunnel) 102 so as to form an inverse flap on the surface of the blood vessel (see Fig. 8B) in the cephalic vein in a foreleg of each dog, and puncture time was measured for the dull needle 1 which was prepared by injection molding of polypropylene according to the present invention (see Fig. 19). For comparison, a conventional puncture needle was used to prepare a puncture route (subcutaneous tunnel) 102 so as to form a forward flap on the surface of the blood vessel (see Fig. 8A), and puncture time was measured for the conventional dull needle (Fig. 2).

Table 4

| Evaluation sample | Puncture time (sec.) |
|---|---|
| Dull needle according to the present invention (Sample 1) | 2.5 ± 1.3 |
| Conventional dull needle (Sample 3) | 8.3 ± 4.5 |

The puncture time of the plastic needle (Sample 1) according to the present invention was shorter than the puncture time of the conventional dull needle (Sample 3). The conventional dull needle 1 was sometimes caught by a wall of the puncture route 102 and could not therefore be smoothly inserted into the puncture hole 101 a. On the other hand, the dull needle 1 according to the present invention (Sample 1) did not face such problems and could make a puncture with ease.

Industrial Applicability

The present invention is useful in reducing damage in part of a puncture route, a blood vessel and the peripheral parts thereof during an operation of puncturing a blood vessel through a puncture route and in improving a needle's ability to search for an optimum contact point in the blood vessel. The present invention suppresses the formation of a pocket, being a potential source of infection, in a shunt of a patient, and therefore provides excellent infection control.

Reference Signs List

1: dull needle (needle)
1a: needle cylinder part
1b: connecting portion between the needle cylinder part and a holding part
2: blood purification treatment device
3: puncture needle (inverse puncture needle for initial puncture)
10: tube
11: connecting part
12: holding part
13: clamp
14: cap
20: inclined end face
20a: tip end
20b: side edge portion
30: opening
40: support table
50: ring gate
51: disc
52: connecting tube
53: ring
100: skin
101: blood vessel
101 a, 111 a: puncture hole
101b, 111b: flap
102: puncture route
103: crust

104: optimum contact point
150: pin gate
200: runner
P: supporting point
F: test force
L: distance between supporting points
K: tip width
T1, T2: arcuate portion

**Claims**

1.  A needle to be inserted into a puncture route extending from skin to a blood vessel under the skin to puncture the blood vessel, wherein:

    the needle is formed in a tubular shape and has an inclined end face at a tip of the needle, the inclined end face being inclined relative to an axis of the needle, a tip end of the inclined end face defining an unsharp edge; the needle has a flexibility of needle elastic modulus nE = 1-900 N, as defined by an equation to be set forth below; a material of the needle is a resin; and
    the needle is manufactured by injection molding using a ring-shaped gate,

    wherein

    $$\text{the needle elastic modulus } nE = (M_2\text{-}M_1) / (\varepsilon_2\text{-}\varepsilon_1),$$

    where:

    $M_1$ is a bending moment under a bending strain of 0.0005 in a three-point bending test;
    $M_2$ is a bending moment under a bending strain of 0.0025 in the three-point bending test;
    $\varepsilon_1$ is a bending strain of 0.0005; and
    $\varepsilon_2$ is a bending strain of 0.0025.

2.  A needle to be inserted into a puncture route extending from skin to a blood vessel under the skin to puncture the blood vessel, wherein:

    the needle is formed in a tubular shape and has an inclined end face at a tip of the needle, the inclined end face being inclined relative to an axis of the needle, a tip end of the inclined end face defining an unsharp edge; a material of the needle is a resin having an elastic modulus in bending of 300-2000 MPa and/or having an elastic modulus in tension of 200-2000 MPa, and/or having a deflection temperature under load (JIS K7191 (ISO 75)) at 0.45 MPa of 70-115°C; and
    the needle is manufactured by injection molding using a ring-shaped gate.

3.  A needle to be inserted into a puncture route extending from skin to a blood vessel under the skin to puncture the blood vessel, wherein:

    the needle is formed in a tubular shape and has an inclined end face at a tip of the needle, the inclined end face being inclined relative to an axis of the needle, a tip end of the inclined end face defining an unsharp edge; the needle has a flexibility of needle elastic modulus nE = 1-900 N as defined by an equation to be set forth below; a material of the needle is a resin;
    the inclined end face of the needle is free from a cut mark formed by post-treatment; and
    the needle has a reduced amount of short shot, flash and gate marks, has more uniform wall thickness, and has smoother inner and outer surfaces, as compared to a needle manufactured by injection molding using a pin gate, wherein

    $$\text{the needle elastic modulus } nE = (M_2\text{-}M_1) / (\varepsilon_2\text{-}\varepsilon_1),$$

where:

$M_1$ is a bending moment under a bending strain of 0.0005 in a three-point bending test;
$M_2$ is a bending moment under a bending strain of 0.0025 in the three-point bending test;
$\varepsilon_1$ is a bending strain of 0.0005; and
$\varepsilon_2$ is a bending strain of 0.0025.

4. A needle to be inserted into a puncture route extending from skin to a blood vessel under the skin to puncture the blood vessel, wherein:

the needle is formed in a tubular shape and has an inclined end face at a tip of the needle, the inclined end face being inclined relative to an axis of the needle, a tip end of the inclined end face defining an unsharp edge; a material of the needle is a resin having an elastic modulus in bending of 300-2000 MPa and/or having an elastic modulus in tension of 200-2000 MPa, and/or having a deflection temperature under load (JIS K7191 (ISO 75)) at 0.45 MPa of 70-115°C; the inclined end face of the needle is free from a cut mark formed by post-treatment; and the needle has a reduced amount of short shot, flash and gate marks, has more uniform wall thickness, and has smoother inner and outer surfaces, as compared to a needle manufactured by injection molding using a pin gate.

5. The needle according to any one of claims 1 to 4, wherein the needle is inserted into the puncture route in which, by inserting a puncture needle with an inclined end face thereof being oriented downward in the puncture route, a puncture mark that opens in an inverted U-shape starting from a back side toward a front side in a puncturing direction is formed in a surface of the blood vessel.

6. The needle according to any one of claims 1 to 5, wherein a wall thickness of an arcuate part of the needle is from 0.02 mm to 0.22 mm.

7. The needle according to any one of claims 1 to 6, wherein the wall thickness of an arcuate portion on a tip end side of the inclined end face is larger than a wall thickness of the remaining arcuate portion, as viewed from a tip side of the needle.

8. The needle according to any one of claims 1 to 7, wherein the tip end of the inclined end face is curved upward as viewed from a lateral side of the needle which is horizontally laid still with the inclined end face being oriented upward.

9. The needle according to any one of claims 1 to 8, wherein right and left side edges leading to the tip end of the inclined end face are each formed linearly as viewed from an upper side of the needle which is horizontally laid still with the inclined end face being oriented upward.

10. The needle according to any one of claims 1 to 9, wherein the needle is integrally molded with a holding part for holding the needle, and a flow path for a liquid such as blood within the holding part is tapered such that an inner diameter of the flow path gradually decreases toward the tip of the needle.

11. The needle according to any one of claims 1 to 10, wherein the tip end of the inclined end face defines an arcuate edge.

12. A manufacturing method of a needle to be inserted into a puncture route extending from skin to a blood vessel under the skin to puncture the blood vessel, the method comprising:

using a ring-shaped gate as a gate for connecting a runner to the needle, the ring-shaped gate being constituted by a ring part arranged on a resin runner side, a disc part arranged on a needle side, and a connecting part connecting the ring part and the disc part; and performing injection molding in which a resin whose pressure is increased inside the ring part and which is allowed to fill the disc part through the connecting part flows into the needle.

# FIG.1

2

20a

20

1

12

10

13

11

14

# FIG.2

# FIG.3A

# FIG.3B

# FIG.4

# FIG.5

# FIG.6

# FIG.7

# FIG.8A

101

INSERTION
DIRECTION X

104

101a

# FIG.8B

101

INSERTION
DIRECTION X

104

111a

# FIG.9

# FIG.10

# FIG.11

110a

110

# FIG.12

20a

1

# FIG.13

# FIG.14

# FIG.15

**FIG.16A**

**FIG.16B**

**FIG.16C**

**FIG.17A**

**FIG.17B**

**FIG.17C**

# FIG.18

1

150

200

# FIG.19

53

52

50

51

1

200

# FIG.20

# FIG.21A

# FIG.21B

FIG.22C

FIG.22A

FIG.22B

## FIG.23

## FIG.24

# FIG.25

# FIG.26

# FIG.27A

CONVENTIONAL CONFIGURATION

ORDINARY NEEDLE 110 FOR INITIAL PUNCTURE

(1) FIRST STEP (PUNCTURE ROUTE CREATION)

101

101b

101a

104

(2) SECOND STEP (AFTER PUNCTURE ROUTE CREATION)

DULL NEEDLE 1

101

# FIG.27B

PRESENT INVENTION

INVERSE PUNCTURE NEEDLE 3 FOR INITIAL PUNCTURE

3

101

111b

111a

104

DULL NEEDLE 1

101

OPPOSITE OPENING DIRECTIONS OF FLAP 101B AND FLAP 111B

## INTERNATIONAL SEARCH REPORT

| International application No. |
| --- |
| PCT/JP2016/051181 |

| A. CLASSIFICATION OF SUBJECT MATTER |
| --- |
| *A61M5/158*(2006.01)i, *A61M1/14*(2006.01)i |

According to International Patent Classification (IPC) or to both national classification and IPC

| B. FIELDS SEARCHED |
| --- |
| Minimum documentation searched (classification system followed by classification symbols)<br>A61M5/158, A61M1/14 |

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)
WPI

| C. DOCUMENTS CONSIDERED TO BE RELEVANT | | |
| --- | --- | --- |
| Category* | Citation of document, with indication, where appropriate, of the relevant passages | Relevant to claim No. |
| Y | JP 2011-250999 A  (Asahi Kasei Kuraray Medical Co., Ltd., Nextier Corp.), 15 December 2011 (15.12.2011), paragraphs [0024] to [0027], [0034]; fig. 1 to 4, 9 & US 2011/0295152 A1 fig. 1 to 4; paragraphs [0118] to [0121], [0128] | 4-11 |
| Y | JP 7-236697 A  (Nippon Zeon Co., Ltd.), 12 September 1995 (12.09.1995), paragraph [0007]; fig. 3(B) (Family: none) | 4-11 |

☐ Further documents are listed in the continuation of Box C.   ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| | |
| --- | --- |
| Date of the actual completion of the international search<br>07 April 2016 (07.04.16) | Date of mailing of the international search report<br>19 April 2016 (19.04.16) |
| Name and mailing address of the ISA/<br>Japan Patent Office<br>3-4-3,Kasumigaseki,Chiyoda-ku,<br>Tokyo 100-8915,Japan | Authorized officer<br><br><br>Telephone No. |

Form PCT/ISA/210 (second sheet) (January 2015)

## INTERNATIONAL SEARCH REPORT

| International application No. |
| --- |
| PCT/JP2016/051181 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☒ Claims Nos.: 1-3, 12
   because they relate to parts of the international application that do not comply with the prescribed requirements to such an extent that no meaningful international search can be carried out, specifically:
   In claim 1, the unit of the needle elastic modulus is N which is a unit of a force, whereas the unit of the right-hand side in the equation that defines the needle elastic modulus is Nm which is a unit of a (bending) moment. (Continued to extra sheet)

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.

PCT/JP2016/051181

Continuation of Box No.II-2 of continuation of first sheet(2)

Further, also the description similarly discloses.
The above-said opinion may be also applied to claim 3.
The "injection molding using a ring-shaped gate" described in claim 2 is unclear.
Ditto for the method for producing a needle described in claim 12.

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009045124 A